# EUROPEAN PATENT SPECIFICATION

(11) **EP 0 854 150 B1**
(45) Date of publication and mention of the grant of the patent: **15.02.2006**
(21) Application number: 97100481.7
(22) Date of filing: 15.01.1997
(51) Int. Cl.: C07K 14/47, A61K 38/04

(54) **Inhibition of binding of complement factor H**
Bindungsinhibition des H-Komplementfaktors
Inhibition de la liaison du facteur H du complément

(43) Date of publication of application: 22.07.1998
(73) Proprietor: Dierich, Manfred Paul, 6020 Innsbruck (AT); Clivio, Alberto, 21030 Orino (VA) (IT); Stoiber, Heribert, 6020 Innsbruck (AT)
(72) Inventor: Dierich, Manfred Paul, 6020 Innsbruck (AT); Clivio, Alberto, 21030 Orino (VA) (IT); Stoiber, Heribert, 6020 Innsbruck (AT)
(74) Representative: Torggler, Paul Norbert

(56) References cited:
- JOURNAL OF BIOLOGICAL CHEMISTRY, vol. 266, no. 25, 5 September 1991, MD US, pages 16847-16853, XP002035067 M K PANGBURN ET AL.: "Localization of heparin-binding site on complement factor H"
- PROCEEDINGS OF THE NATIONAL ACADEMY OF SCIENCES OF USA, vol. 93, no. 20, 1 October 1996, WASHINGTON US, pages 10996-11001, XP002035068 A K SHARMA & M K PANGBURN: "Identification of three physically and functionally distinct binding sites for C3b in human complement factor H by deletion mutagenesis"
- AIDS RESEARCH AND HUMAN RETROVIRUSES, vol. 11, no. 8, November 1995, MARY ANN LIEBERT INC., NEW YORK, USA, pages 971-980, XP002035069 C PINT¹R ET AL.: "HIV glycoprotein 41 and complement factor H interact with each other and share functional as well as antigenic homology"
- AIDS RESEARCH AND HUMAN RETROVIRUS, vol. 11, no. 5, May 1995, MARY ANN LIEBERT INC., NEW YORK, USA, pages 577-588, XP002035070 C PINT¹R ET AL.: "Direct interaction of complement factor H with the C1 domain of HIV type I glycoprotein 120 "
- JOURNAL OF IMMUNOLOGY, vol. 157, no. 12, 15 December 1996, BALTIMORE US, pages 5422-5427, XP002035071 T K BLACKMORE ET AL.: "Identification of a heparin binding domain in the seventh short consensus repeat of complement factor H"
- STOIBER ET AL.: "Efficient Destruction of Human Immunodeficiency Virus in Human Serum by Inhibiting the Protective Action of Complement Factor H and Decay Accelerating Factor (DAF, CD55)" J. EXP. MED, vol. 183, 1 January 1996 (1996-01-01), pages 307-310,

## Description

### Background of the Invention

### (1) Field of the Invention

This invention relates generally to peptide chains used to block binding sites on pathogens and more particularly to peptide chains used to block binding sites for Complement Factor H (CFH) on pathogens such as the Human Immunodeficiency Virus (HIV).

### (2) Description of Related Art

Various pathogens, like HIV, have evolved the ability to escape from complement-mediated lysis. See, e.g., Marschang P. Sodroski J., Wurzner R. and Dierich M.P., "Decay-accelerating Factor (CD55) Protects Human Immunodeficiency Virus Type 1 From Inactivation by Human Complement," Eur. J. Immunol. 25: 285-90 (1995) and Horstmann, R.D., Sievertsen, H.J., Knobloch, J. and Fischetti, V.A., "Antiphagocytic Activity of Streptococcal M Protein: Selective Binding of Complement Control Protein Factor H," Proc. Nat'l. Acad. Sci. U.S.A. 85: 1657-1661 (1988). The complement system is part of the immune system. Its main functions are the opsonisation of micro-organisms to allow more efficient phagocytosis and complement-mediated lysis of pathogens. To turn down the activity of complement and to protect host cells from unspecific damage, regulators of complement activity (RCA's) are required. Among this family of negative regulators is CFH, a very abundant plasma protein. CFH has been shown to down-regulate the alternative pathway of complement activation by directly affecting the formation and stability of C3 and C5 convertase and by acting as a cofactor for the cleavage of C3b into its inactive form iC3b as described by Whaley, K. and Ruddy, S., "Modulation of the Alternative Complement Pathway by β1H Globulin," J. Exp. Med. 144: 1147 (1976) and Pangburn, M.K., Schreiber and Eberland, H.J. Muller, "Human Complement C3b Inactivator: Isolation, Characterization and Demonstration of an Absolute Requirement for the Serum Protein β1H for Cleavage of C3b and C4b in Solution," J. Exp. Med. 146:257 (1977). Responsible for these effects is a functional site located in the first 5 short consensus repeats (SCR 1-5) of CFH as described in Alsenz, J., Lambris, J.D., Schulz, T.F. and Dierich, M.P., "Localization of the Complement Component C3b-binding Site and the Cofactor Activity for Factor I in the 38-kDa Tryptic Fragment of Factor H," Biochem. J. 224: 389 (1984) and Gordon, D.L., Kaufman, R.M., Blackmore, T.K., Kwong, J. and Lublin, D.M., "Identification of Complement Regulatory Domains in Human Factor H," J. Immunol. 155: 348 (1995). An interaction site for polyanionic molecules has been mapped in SCR 13. Pangburn, M.K., Atkinson, M.A. and Meri, S., "Localization of the Heparin-binding Site on Complement Factor H," J. Biol. Chem. 266: 16847 (1991). For example, SCR 13 has been shown to bind streptococcal protein M and is responsible for protection of M+ strains from complement-mediated bactericidal activity. See Horstmann et al. article, above. Recently, additional binding regions for negatively-charged particles in SCR 7 have also been described. Blackmore, T. and Gordon, D., "SCR 7 is a Major Heparin/Sialic Acid Binding Site of Complement Factor H," J. Mol. Immunol. 33 (S1): 15 (1996). It is believed that SCR 7 is a potential binding site for host cells.

Applicants have recently shown the CFH interacts with specific sites on the envelope of HIV. Specifically, envelope glycoproteins, gp120 and gp41, have been demonstrated to be binding sites for CFH. See Stoiber, H., Ebenbichler, C., Schneider, R., Janatova, J. and Dierich, M.P., "Interaction of Several Complement Proteins with gp120 and gp41, the Two Envelope Glycoproteins of HIV-1," AIDS 9:19 (1995) and Pinter, C., Siccardi, A.G., Longhi, R. and Clivio, A., "Direct Interaction of Complement Factor H with the C1 Domain of HIV Type 1 Glycoprotein 120," AIDS Research & Human Retroviruses 11: 577 (1995). Incubation of free HIV or HIV-infected cells with sera that has been depleted of CFH (NHS^{CFH-}) results in the complement-mediated virolysis of primary HIV isolates and the killing of HIV-infected cells. Re-titration of CFH to NHS^{CFH-} reconstitutes the resistance of HIV against complement-mediated-destruction. See Stoiber, H., Pinter, C., Siccardi, A.G., Clivio, A. and Dierich, M.P., "Efficient Destruction of Human Immunodeficiency Virus in Human Serum by Inhibiting the Protective Action of Complement Factor H and Decay Accelerating Factor (DAF, CD55)," J. Exp. Med. 183: 307 (1996).

In addition, Applicants have shown that the antibody 2F5, described by Katinger et al. (See WO95/07354 A1) interacts with a CFH binding site in gp41. From this information, the Applicants hypothesized that one of the HIV-neutralizing effects of this antibody is due to the inhibition of CFH interaction with gp41, resulting in complement-mediated lysis of the virus.

The present invention is based on the assumption that if the hypothesis described above is correct, it is not necessary to use an antibody for specifically blocking the binding sites on pathogens. Using a molecule which is able to block sites on pathogens which act as binding sites for CFH should have the same or even more promising effects.

### Summary of the Invention

It is accordingly an object of the invention to eliminate the need for antibody-mediated pathogen destruction. It is a further object of the invention to provide a pathogen specific peptide chain that binds to the sites that would otherwise be available to bind CFH so that CFH-mediated lysis of pathogens is not prevented.

It is a still further object of the invention to provide a peptide chain that does not have the active sites of CFH and does not have the binding sites on CFH that bind to host cells to regulate complement activity.

By screening with specific peptides, Applicants have discovered that CFH binding on pathogens can be inhibited so that complement-mediated destruction of the pathogens is induced. The Applicants have discovered that binding inhibition can be accomplished with peptides containing less than 100 amino acids. The amino acid chains, SEQ. ID NO: 1, were derived from the SCR 13 region of CFH. Whole CFH has been modified so that the areas involved in mediating the interaction with pathogens are conserved while the areas responsible for inhibition of complement-mediated lysis are deleted. Additionally, areas believed to be involved in binding to host cells such as SCR7 have also been deleted to provide a pathogen-specific peptide chain.

Such pathogen-specific peptide chains can be synthesized in a variety of ways including mutation and expression of mutated CFH DNA. Using known peptide synthesis procedures, it is even possible to use cyclic peptides or introduce D-amino acids which can result in higher resistance of the peptide chain to degradation by human serum.

By screening peptide and pseudopeptide libraries, the invention offers the possibility to detect in addition to the described peptides, other molecules which interfere with CFH binding on various pathogens. The effect of those substances can be easily checked by incubating pathogen which are resistant to human serum with antibodies specific against the pathogen, serum and peptides or pseudopeptides followed by testing and analyzing for the presence of complement-mediated lysis of the pathogen.

### Brief Description of the Drawings

FIG.1 is a depiction of SEQ. ID NO: 1 and modified variants thereof resulting from selective deletions.
FIG. 2 depicts the results of the binding of biotin-labelled peptide A to HIV-derived peptides with the values being expressed as absorbance at 492 nm.
FIG. 3 is a bar graph showing the effect of SEQ. ID NO: 1 on complement-dependent cytotoxicity induced on HIV-infected cells.

### Description of the Preferred Embodiments

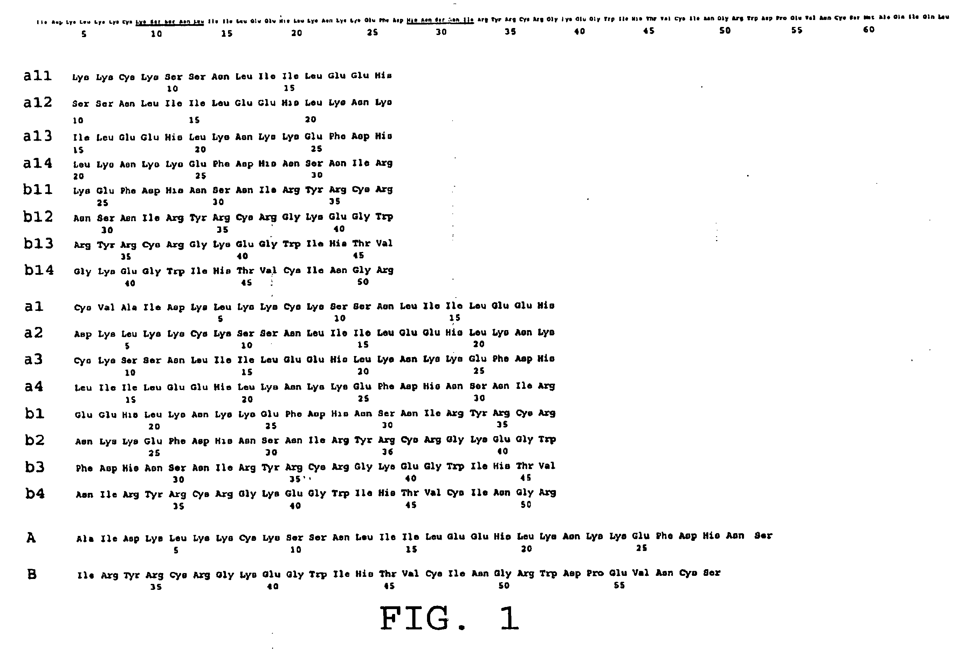

As shown in FIG. 1, peptides A and B were synthesized with the step-wise solid-phase method described in Merrifield, R.B., "Solid-phase Peptide Synthesis," Science 232: 341 (1986), on an Abi 433A Peptide Synthesizer (Applied Biosystems, Foster City, CA) at a scale of 0.1 mmole. The overlapping peptides, 17 mer in length, including Gly at the amino terminus as the spacer and Glycyl-Tyrosine at the carboxy terminus to enable spectrophotometric quantitation were synthesized manually at a scale of 0.02 mmoles on NovaSyn TGA resin using chemical protocols based on the Fmoc strategy, Knorr, R., Trzeciak, A., Bannwarth, W. and Gillessen, D., "New Coupling Reagents in Peptide Chemistry," Tetrahedron Lett. 30:1927 (1989), and in situ amino acid activation, Carpino, L.A. and Han, G.Y., "The 9-Fluorenylmethoxycarbonyl Amino Protecting Group," J. Org. Chem. 37:3404 (1972). The apparatus for the manual, small scale simultaneous multiple peptide synthesis was essentially that described by Knapp in Knapp, D.R., Oatis, J.E. and Papac, D.I., "Small-scale Manual Multiple Peptide Synthesis System," Int. J. Peptide Protein Res. 42:259 (1972). Resins, N-α-9-fluorenylmethoxycarbonyl amino acids (side chain protected) and activating reagents such as 2-(1 H-Benzotriazole-1-yl)1,1,3,3-tetramethyluronium tetrafluoroborate (HBTU) and N-Hydroxybenzotriazole (HOBt) were purchases from Novabiochem (Laufelfingen, Switzerland). A ten-fold excess of active reagent over the amino terminal content of the resin was used both in the automatic and in the manual peptide synthesis. After assembly, the completed peptides were fully de-protected and removed from the solid support according to the cleavage method suggested by King in King, D.S., Fields, C.G. and Fields, G.B., "A cleavage Method Which Minimizes Side Reactions Following Fmoc Solid Phase Peptide Synthesis," Int. J. Pept. Protein. Res. 36: 255 (1990). The free peptides were repeatedly lyophilized from water and analyzed by reverse phase liquid chromatography (RF-HPLC), to assess their homogeneity. Amino acid analysis was carried out on all the peptide preparations before use. The peptides of FIG. 1 were used in the immunometric assay both before and after purification.

Synthetic peptides with different lengths and overlapping patterns of the whole SEQ. ID NO: 1 were used as shown in FIG. 1. "Mutagenized" peptides using alanine as a substitute for the naturally occurring amino acids in critical positions of SCR 13 of CFH were also produced. Finally, amino acids contained in the known active regions of CFH were scrambled to check the sequence dependence and specificity of the peptide biologic activity.

Regarding the HIV envelope peptides, only #SA2 (His Glu Asp Ile Ile Ser Leu Trp Asp Gln Ser Leu Lys Pro Cys, Env. 105-119) of the gp120-derived peptides, described by Pinter in the AIDS Research & Human Retroviruses article rgferenced above as being the target of CFH binding, was used in this study to reproduce the CFH-gp120 interaction. Peptides #18 (Asn Lys Ser Leu Glu Gln Ile Val Asn Asn Met Thr Trp Met Glu Trp Asp Arg Glu Ile Asn Asn Thr Tyr THr Ser, Env. 621-645) and #12 (Leu Ile Glu Glu Ser Gln Asn Gln Gln Glu Lys Asn Glu Gln Glu Leu Leu Glu Leu Asp Lys Trp Ala Ser, Env. 650-673) of the gp41-derived peptides described in Pinter, C., Siccardi, A.G., Lopalco, L., Longhi, R. and Clivio, A., "HIV Glycoprotein 41 and Complement Factor H Interact with Each Other and Share Functional as well as Antigenic Homology," AIDS Research & Human Retroviruses 11:971 (1995) as being the targets of CFH binding were used to reproduce the CFH-41gp interaction. Peptide #53 (Ala Arg Gln Leu Leu Ser Gly Ile Val Gln Gln Gln Asn Asn Leu Leu Arg Ala Ile Glu Ala Gln Gln His Leu, Env. 546-570) from the gp41 ectodomain which has shown no CFH binding was used as a negative control.

Cell lines and tissue culture techniques 8E5 cells which are chronically infected with a polymutant of LAV, uninfected CD4+CEM cells, HIV-IIIb-infected and uninfected H9 cells were obtained from the American Type Culture collection (ATCC). Cells adapted to grow in a protein-free medium supplemented with 4mM L-glutamine and in the absence of antibiotics (S-2897, SIGMA) as described, were maintained in a humidified CO₂ incubator (NAPCO). Cell density was always kept between 8x10⁵ and 1.5x10⁶ cells/ml.

CFH was purified as described by Pinter et al., AIDS Research & Human Retroviruses 11: 971. Briefly, normal human plasma was desalted, diluted 1:4 in buffer A (20mM Tris-HCl pH 8.5), and fractionated by anion exchange HPLC (MA7P BioRad, Milan, Italy), and hydrophobic interaction (HI) HPLC (BioRad). The last peak eluting from the HI column contained only CFH and albumin. The albumin was removed by vacuum dialysis using 100 kDa cut off membranes and subsequent concentration. This fraction contained pure CFH as determined by one- and two-dimensional SDS-polyacrylamide gel electrophoresis.

Synthetic peptides and recombinant proteins were dissolved in Carbonate-bicarbonate buffer, 50mM, pH 9.2 at 50 µg/ml (micrograms per milliliter) and 10 µg/ml, respectively, distributed in 100 µl aliquots into flat-bottomed 96-well ELISA plates (Greiner, Germany) and left at 37°C for 3 hours. The plates were then washed three times in phosphate-buffered saline (PBS) and the wells were overcoated with a 3% (w/v) solution of bovine serum albumin (BSA) in PBS. The CFH source (either from fresh plasma or from plasma subjected to subsequent purification steps), was diluted to the appropriate concentration (1mg/ml) in PBS-BT (= PBS containing 1% BSA and 1% Tween 20), pH 7.0 and exposed to the solid phase for one hour at 37°C. After washing in PBS+1% Tween 20 (= PBS-T), bound CFH was detected by the use of specific, biotin-labelled MAb 5H5, Pinter et al., AIDS Research & Human Retroviruses 11: 971, and peroxidase-labelled streptavidin (Sigma, Italy). The reaction was developed with o-phenylene-diamine/H₂O₂ for fifteen minutes and then stopped by the addition of 4N H₂SO₄. Absorbance was detected at 492 µm with a MT450 microplate reader (BioRad).

Along with the above-described assay, a preincubation assay was also conducted with peptides. The general features of this assay are essentially the same as those described for the CFH binding assay above, except that before the addition of CFH, the solid phases were preincubated for one hour with increasing concentrations of putative competitors. Peptides used as competitors were dissolved in PBS-BT with a pH of 7.0. The solid phases were washed in PBS-T and CFH was detected with the biotin-labelled MAb 5H5 as previously described.

In these experiments, the competitors were added to the solid phases simultaneously with CFH. Peptides were dissolved in PBS-BT with a pH of 7.0. Peptide concentrations ranged from 0 to 50 µg/ml. Bound CFH was detected as in the previous assay. Heparin (Sodium salt, Fluka, Germany) was dissolved in PBS-T at 50 mg/ml as a stock solution and then further diluted in the assays.

To test for induction of complement-dependent, HIV-specific cytolysis by SEQ. ID NO: 1 peptides, a complement-dependent cytolytic assay similar to the one described in Stoiber et al., J. Exp. Med. 183: 307, was used to check the possibility of inhibiting the HIV-CFH interaction with the addition of exogenous reagents. To construct this assay, chronically HIV-infected 8E5 cells and uninfected CEM cells were preincubated with antibodies from AIDS sera (AIDS Ig) or from normal human serum (NHS Ig) and incubated with human serum (1:10 diluted) as the complement source in the presence, or in the absence, of exogenously added, putative inhibitors of the CFH-HIV interaction. Complement-dependent cytolysis (CDC) was detected by counting viable cells in 24-well short term (lb-exposure) cultures. Serum depleted of CFH was used as the positive CDC control.

Since three HIV envelope-derived peptides have been previously identified as targets of CFH binding, these peptides (#SA2, #18 and #12) were fixed on ELISA plates and used as CFH "catchers," in the absence of, and in the presence of, putative competitors. Two relatively long peptides (A and B as shown in FIG. 1), which comprise the majority of SEQ. ID NO: 1, the sequence of which was derived from the Swiss-Prot database, were used as a first glance analysis aimed at restricting the region of SEQ. ID NO: 1 which interacts with HIV. Peptide A is a 30 residue peptide representing the N-proximal region of SEQ. ID NO: 1. Peptide B is a 28-mer spanning the rest of the sequence, with the exception of the Asparagine in position 775. Both peptides were used to compete with the interaction of CFH with the HIV target sequences. Heparin was used as the positive competition control. The primary reason SEQ. ID NO: 1 has been selected for testing is due to previous work described in Pinter et al., AIDS Research & Human Retroviruses 11:577, in which it was found that heparin inhibited the binding of CFH to HIV and to the target peptides.

Peptide A competed very efficiently with the binding of CFH to the three envelope peptides used. This result suggests that the same region of SCR 13 is involved in the binding to the target sequences in gp120 and gp41. The inhibition was due to the interaction of peptide A with the solid phase. Preincubation of the target peptides with peptide A before performing the assay gave the same effect as simultaneous competition. Peptide B had almost no effect. Heparin competed only when incubated simultaneously with CFH, as expected, but was inactive in preincubation experiments.

To better localize the CFH binding site, more detailed competition assays were performed with the whole set of short overlapping 14-mer and 21-mer synthetic peptides of SEQ. ID NO: 1 as shown in FIG. 1. Regarding the 21-mer peptides, maximum competition was demonstrated by peptides a2, b1 and b2 which define two regions of SCR 13 that are involved in binding to the HIV envelope peptides. Regarding the 14-mer peptides, b11 and b12 retained all the inhibitory capacity of their corresponding 21-mer peptide chains, b1 and b2. In contrast, the short peptide chains corresponding to the longer 21-mer a2 peptide chain, did not demonstrate competition with the binding of CFH to the HIV envelope peptides thereby indicating with regard to these short peptide chains, that the region of interaction may be more extended, or that a particular peptide conformation is required for binding.

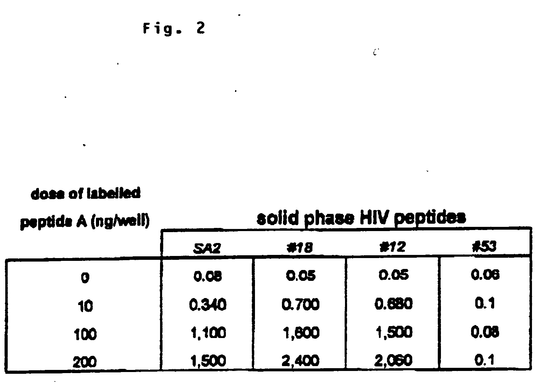

Peptide A, labelled with biotin during synthesis as previously described, was used to confirm the interaction observed in the competition assays. The results are shown in FIG. 2. This peptide, being used as a tracer, was able to bind efficiently to the gp120 peptide #SA2 and to the gp41 peptides #12 and #18. This interaction was competed by peptide A itself, by the envelope peptides #SA2, #12 and #18 but not by peptide #53. Therefore, the three HIV-derived peptides seem to share functional homology with respect to the interaction with CFH since they all bind to the same region on SEQ. ID NO: 1. Peptide b11 has been shown to inhibit the binding of peptide A and the purified CFH molecule to all three HIV-derived target peptides.

To better define the regions of SCR 13 responsible for the binding of HIV, the peptide b11 was modified during synthesis as follows:
a) Alanine was substituted for the naturally occurring amino acids;
b) The putative active sequence, His Asn Ser Asn Ile, was scrambled and the flanking sequences were left unchanged;
c) The putative active sequence of region I(Lys Ser Ser Asn Leu) was grafted into peptide b11 in substitute of the original His Asn Ser Asn Ile core;
d) The two amino acids flanking the active box of peptide b11 and the active box of the "grafted" peptide were substituted with cysteines and covalently closed.

All of these peptides were used to compete with CFH binding to the HIV target peptides. The results of an "alanine" scan indicate that mutations within the core sequence of peptide b11 totally abolish the ability of the peptides to compete with CFH binding. The data also indicate that the flanking sequences also contribute to the peptide activity since mutations in these regions decrease the competing capacity of the resulting peptides.

The scrambled peptide suggests that the sequences flanking the central box do not participate in the binding to HIV; this peptide contains unmodified flanking sequences and its core contains the naturally occurring amino acids randomly sorted. It is clear that the core must contain the natural sequence to be able to compete with CFH binding. It is also clear that the flanking sequences are unable to compete by themselves. The core of region I (Lys Ser Ser Asn Leu) and that of region II (His Asn Ser Asn Ile) were shown to be functionally homologous. When the core of region I was grafted into the flanking sequences of peptide b11 (FIG. 1), partial competition was readily observed.

¹²⁵I-labelled CFH was used to show that in addition to binding to recombinant gp41, Stoiber, H., Schneider, R., Janatova, J. and Dierich, M.P., "Human Complement Proteins C3b, C4b, Factor H and Properdin React with Specific Sites in gp120 and gp41, the Envelope Proteins of HIV-1," Immunobiology 193: 98 (1995) and Pinter et al., AIDS Research & Human Retroviruses 11: 971, and to envelope-derived synthetic peptides in ELISA, CFH is also able to interact with envelope proteins at the surface of HIV-infected cells. A significant difference in the efficiency of CFH binding between infected compared to uninfected cells was observed. As a control, the binding of ¹²⁵I-labelled CFH was inhibited using cold CFH. The ELISA assays with immobilized rsgp41 gave similar results. The standard deviation in these assays was below 15%.

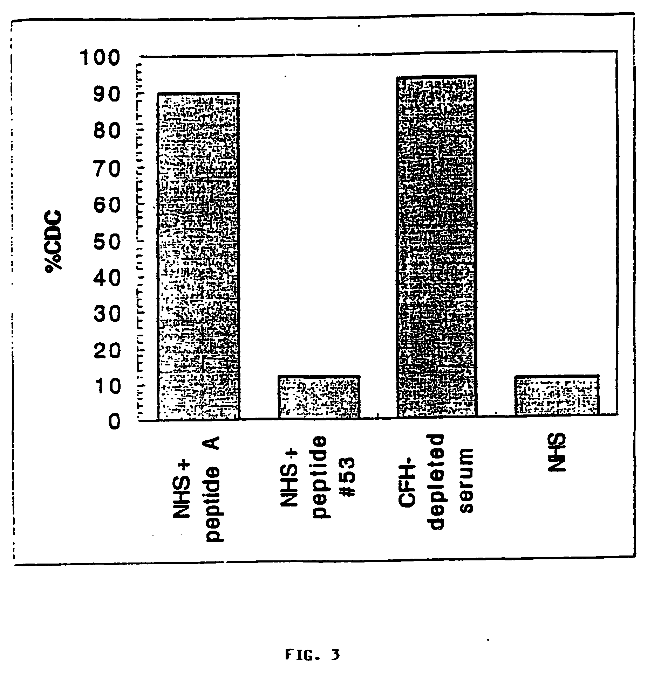

Based on the results of the binding assays on intact cells, and on the Applicants' previous observation that CFH depletion results in HIV-specific complement-dependent cytolysis, Stoiber et al., J. Exp. Med. 183: 307, the possibility of blocking the intrinsic HIV-infected cell resistance to complement lysis by the addition of exogenous inhibitors of the CFH-HIV envelope protein interaction was checked. Peptide A, as a representative of CFH inhibitors, was used as the competitor in the CDC assay described above and in Stoiber et al., J. Exp. Med. 183: 307. As shown in FIG. 3, the presence of peptide A in the incubation mixture resulted in highly efficient CDC induction which was similar to that of the CFH-depleted control.

## Claims

1. A process for selecting candidate substances for the use as reagents for the treatment of diseases caused by pathogens resistant to human serum, the process including the addition of peptides containing less than one hundred amino acids to a mixture comprising the pathogen, serum and antibodies specific for the pathogen and measuring any resulting increase of complement-mediated cytotoxicity due to said addition.

2. A process according to claim 1, wherein the pathogens are known to be destroyed by CFH depleted serum.

3. A process according to claim 2, wherein the pathogen is HIV.

4. A process according to claim 2, wherein the pathogen is a streptococcus.

5. A reagent for the treatment of diseases caused by HIV, having in common with CFH a peptide chain of less than 100 amino acids containing regions of SCR 13 of CFH (SEQ. ID NO:1) including regions of Lys Ser Ser Asn Leu and His Asn Ser Asn Ile of CFH.

6. A reagent according to claim 5, wherein said regions of Lys Ser Ser Asn Leu and His Asn Ser Asn Ile of CFH are parts of a cyclic molecule.

## Revendications

1. Procédé de sélection de substances candidates à utiliser en tant que réactifs pour le traitement de maladies causées par des agents pathogènes résistant au sérum humain, le procédé comprenant une addition de peptides contenant moins de cent acides aminés à un mélange comprenant l'agent pathogène, du sérum et des anticorps spécifiques de l'agent pathogène, et une mesure de toute augmentation résultante , de la cytotoxicité induite par le complément due à ladite addition.

2. Procédé selon la revendication 1, dans lequel les agents pathogènes sont connus pour être détruits par du sérum appauvri en CFH.

3. Procédé selon la revendication 2, dans lequel l'agent pathogène est le VIH.

4. Procédé selon la revendication 2, dans lequel l'agent pathogène est un streptocoque.

5. Réactif pour le traitement de maladies causées par le VIH, ayant en commun avec le CFH une chaîne peptidique de moins de 100 acides aminés contenant des régions SRC 13 de CFH (SEQ. ID. NO : 1) comprenant des régions Lys Ser Ser Asn Leu et His Asn Ser Asn Ile de CFH.

6. Réactif selon la revendication 5, dans lequel lesdites régions Lys Ser Ser Asn Leu et His Asn Ser Asn Ile de CFH font partie d'une molécule cyclique.

## Patentansprüche

1. Verfahren zur Auswahl von Substanzen, welche für die Verwendung als Reagenzien für die Behandlung von Krankheiten in Frage kommen, welche von gegenüber menschlichem Serum resistenten Pathogenen verursacht werden, wobei Peptide, welche weniger als hundert Aminosäuren umfassen, einer Mischung zugeführt werden, welche das Pathogen. Serum und für das Pathogen spezifische Antikörper enthält und wobei eine allfällige Erhöhung durch Komplement vermittelter Zytotoxizität, welche auf diese Hinzufügung zurückgeht, gemessen wird.

2. Verfahren nach Anspruch 1, **dadurch gekennzeichnet, dass** die Pathogene bekanntermaßen durch CFH-freies Serum zerstört werden.

3. Verfahren nach Anspruch 2, **dadurch gekennzeichnet, dass** das Pathogen HIV ist.

4. Verfahren nach Anspruch 2, **dadurch gekennzeichnet, dass** das Pathogen ein Streptokokkus ist.

5. Reagens zur Behandlung von Krankheiten, welche durch HIV verursacht werden, **dadurch gekennzeichnet, dass** es mit CFH eine Peptidkette mit weniger als 100 Aminosäuren gemein hat und Bereiche von SCR 13 von CFH (SEQ. ID NO.1) einschließlich Bereichen von Lys Ser Ser Asn Leu und His Asn Ser Asn Ile von CFH enthält.

6. Reagens nach Anspruch 5, **dadurch gekennzeichnet, dass** die Bereiche von Lys Ser Ser Asn Leu und His Asn Ser Asn Ile von CFH Teile eines zyklischen Moleküls sind.
